(19)

Europäisches
Patentamt
European
Patent Office
Office européen
des brevets

(11) **EP 3 736 528 B1**

(12) **EUROPEAN PATENT SPECIFICATION**

(45) Date of publication and mention
of the grant of the patent:
**17.05.2023 Bulletin 2023/20**

(21) Application number: **19173666.9**

(22) Date of filing: **09.05.2019**

(51) International Patent Classification (IPC):
**G01B 7/24** *(2006.01)* **A61B 5/113** *(2006.01)*
**A61B 5/00** *(2006.01)*

(52) Cooperative Patent Classification (CPC):
**G01B 7/24; A61B 5/1135; A61B 5/6805;**
A61B 2562/0261

(54) **A STRAIN SENSOR AND METHODS AND APPARATUSES FOR USING A STRAIN SENSOR**

DEHNUNGSSENSOR UND VERFAHREN UND VORRICHTUNGEN ZUR VERWENDUNG EINES
DEHNUNGSSENSORS

CAPTEUR DE CONTRAINTE ET PROCÉDÉS ET APPAREILS POUR L'UTILISATION D'UN
CAPTEUR DE CONTRAINTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**11.11.2020 Bulletin 2020/46**

(73) Proprietor: **Nokia Technologies Oy
02610 Espoo (FI)**

(72) Inventors:
• **SASSI, Ugo
 Cambridge, Cambridgeshire CB3 0FA (GB)**
• **RADIVOJEVIC, Zoran
 Cambridge, Cambridgeshire CB3 0FA (GB)**
• **DERRICK, Philip
 Cambridge, Cambridgeshire CB3 0FA (GB)**

• **BRUNA, Matteo
 Cambridge, Cambridgeshire CB3 0FA (GB)**

(74) Representative: **Swindell & Pearson Limited
48 Friar Gate
Derby DE1 1GY (GB)**

(56) References cited:
**US-A- 4 890 084 US-A1- 2017 176 167**

• **Ok Sun ET AL: "Application of a Textile-based
Inductive Sensor for the Vital Sign Monitoring",
J Electr Eng Technol, 6 September 2014
(2014-09-06), page 742, XP055145258, DOI:
10.5370/JEET.2014.9.6.742 Retrieved from the
Internet:
URL:http://www.jeet.or.kr/LTKPSWeb/uploadf
iles/be/201409/230920141636103597500.pdf
[retrieved on 2014-10-08]**

## Description

TECHNOLOGICAL FIELD

Examples of the present disclosure relate to a strain sensor and methods, apparatuses and computer programs for using a strain sensor. Some examples relate to a strain sensor and methods, apparatuses and computer programs for using a strain sensor in wearable electronic devices.

BACKGROUND

Wearable electronic devices comprise electronic components which can be integrated within the fabric of an article of clothing or strap or other suitable means. In examples, the electronic components need to be comfortable, water and sweat proof and washable.

US4890084 (D1) relates to an inductance strain gauge. According to D1, side-by-side inductor coils are wound in a single layer around a thin flat tubular frame. In D1, the frame has a closed end portion and is mounted on an insulative base sheet. According to D1 an elongated thin magnetic core is cantilevered from a insulative connecting rod and normally is positioned inside the frame approximately centered between the two coils. In D1, the end portion of the connecting rod remote from the frame is secured to the base sheet. According to D1, the base sheet can be applied to a specimen to be stress tested and is formed to withstand elongation and compression. In D1, lengthwise sliding of movement of the magnetic core in the coil frame due to stress applied to the specimen indicates the strain of the specimen.

BRIEF SUMMARY

The scope of protection sought for various embodiments of the invention is set out by the independent claims. The examples and features, if any, described in this specification that do not fall under the scope of the independent claims are to be interpreted as examples useful for understanding various embodiments of the invention.

According to various, but not necessarily all, embodiments there is provided strain sensor comprising:

> a stretchable substrate comprising a plurality of layers;
> non-stretchable magnetic material fastened to at least one of the plurality of layers; and
> at least one coil of conductive thread wound through the substrate to mechanically interconnect the plurality of layers, wherein at least a portion of the at least one coil of conductive thread is located around at least a portion of the non-stretchable magnetic material.

In some but not necessarily all examples, the strain sensor comprises at least one fastener fastening the non-stretchable magnetic material to at least one of the plurality of layers.

In some but not necessarily all examples, the fastener comprises at least one of glue and one or more stitches.

In some but not necessarily all examples, the non-stretchable magnetic material comprises a rounded tip.

In some but not necessarily all examples, the non-stretchable magnetic material comprises at least one magnetic strip and/or at least one magnetic fibre.

In some but not necessarily all examples, the stretchable substrate comprises a first non-magnetic, non-conductive layer and a second non-magnetic, non-conductive layer, and the first non-magnetic, non-conductive layer and the second non-magnetic, non-conductive layer are the outer layers of the substrate.

According to various, but not necessarily all, embodiments there is provided a wearable electronic device comprising a strain sensor as described herein.

According to various, but not necessarily all, embodiments there is provided a method of using a strain sensor, the method comprising:

> measuring the inductance of a strain sensor, the strain sensor comprising:
> a stretchable substrate comprising a plurality of layers;
> non-stretchable magnetic material fastened to at least one of the plurality of layers; and

at least one coil of conductive thread wound through the substrate to mechanically interconnect the plurality of layers, wherein at least a portion of the at least one coil of conductive thread is located around at least a portion of the non-stretchable magnetic material; and the method comprising:
determining a change in the inductance of the strain sensor.

In some but not necessarily all examples, the method comprises determining at least one physiological measurement of a subject based, at least in part, on the determined change in inductance.

In some but not necessarily all examples, the at least one physiological measurement comprises at least one of breathing rate, heart rate, heart rate variability, blood pressure and posturing of a body part.

In some but not necessarily all examples, determining a change in the inductance of the strain sensor comprises monitoring a variation of the inductance of the strain sensor over time.

In some but not necessarily all examples, the method comprises causing provision of at least one output based on, at least in part, the determined change in the inductance of the strain sensor.

In some but not necessarily all examples, the strain sensor comprises at least one fastener fastening the non-stretchable magnetic material to at least one of the plurality of layers.

In some but not necessarily all examples, the fastener comprises at least one of glue and one or more stitches.

In some but not necessarily all examples, the non-stretchable magnetic material comprises a rounded tip.

In some but not necessarily all examples, the non-stretchable magnetic material comprises at least one magnetic strip and/or at least one magnetic fibre.

In some but not necessarily all examples, the stretchable substrate comprises a first non-magnetic, non-conductive layer and a second non-magnetic, non-conductive layer, and the first non-magnetic, non-conductive layer and the second non-magnetic, non-conductive layer are the outer layers of the substrate.

In some but not necessarily all examples the strain sensor is comprised in a wearable electronic device.

According to various, but not necessarily all, embodiments there is provided an apparatus comprising: means for measuring the inductance of a strain sensor, the strain sensor comprising:

> a stretchable substrate comprising a plurality of layers;
> non-stretchable magnetic material fastened to at least one of the plurality of layers; and

at least one coil of conductive thread wound through the substrate to mechanically interconnect the plurality of layers, wherein at least a portion of the at least one coil of conductive thread is located around at least a portion of the non-stretchable magnetic material; and the apparatus comprising:
means for determining a change in the inductance of the strain sensor.

In some but not necessarily all examples, the apparatus comprises means for determining at least one physiological measurement of a subject based, at least in part, on the determined change in inductance.

In some but not necessarily all examples, the at least one physiological measurement comprises at least one of breathing rate, heart rate, heart rate variability, blood pressure and posturing of a body part.

In some but not necessarily all examples, determining a change in the inductance of the strain sensor comprises monitoring a variation of the inductance of the strain sensor over time.

In some but not necessarily all examples, the apparatus comprises means for causing provision of at least one output based on, at least in part, the determined change in the inductance of the strain sensor.

In some but not necessarily all examples, the strain sensor comprises at least one fastener fastening the non-stretchable magnetic material to at least one of the plurality of layers.

In some but not necessarily all examples, the fastener comprises at least one of glue and one or more stitches.

In some but not necessarily all examples, the non-stretchable magnetic material comprises a rounded tip.

In some but not necessarily all examples, the non-stretchable magnetic material comprises at least one magnetic strip and/or at least one magnetic fibre.

In some but not necessarily all examples, the stretchable substrate comprises a first non-magnetic, non-conductive layer and a second non-magnetic, non-conductive layer, and the first non-magnetic, non-conductive layer and the second non-magnetic, non-conductive layer are the outer layers of the substrate.

In some but not necessarily all examples the apparatus and/or strain sensor is comprised in a wearable electronic device.

According to various, but not necessarily all, embodiments there is provided a system comprising the apparatus as described in any preceding paragraph and a strain sensor as described in any preceding paragraph.

According to various, but not necessarily all, embodiments of the invention there is provided a system comprising an apparatus as described herein and a strain sensor as described herein.

According to various examples useful to understand the invention there is provided a computer program comprising program instructions for causing an apparatus to perform at least the following:

> monitoring the inductance of a strain sensor, the strain sensor comprising:
> a stretchable substrate comprising a plurality of layers;
> non-stretchable magnetic material fastened to at least one of the plurality of layers; and

at least one coil of conductive thread wound through the substrate to mechanically interconnect the plurality of layers, wherein at least a portion of the at least one coil of conductive thread is located around at least a portion of the non-stretchable magnetic material; and to perform:
determining a change in the inductance of the strain sensor.

In some but not necessarily all examples, the computer program comprises program instructions for causing an apparatus to perform determining at least one physiological measurement of a subject based, at least in part, on the determined change in inductance.

In some but not necessarily all examples, the at least one physiological measurement comprises at least one of breathing rate, heart rate, heart rate variability, blood pressure and posturing of a body part.

In some but not necessarily all examples, determining a change in the inductance of the strain sensor comprises monitoring a variation of the inductance of the strain sensor over time.

In some but not necessarily all examples, the computer program comprises program instructions for causing an apparatus to perform causing provision of at least one output based on, at least in part, the determined change in the inductance of the strain sensor.

In some but not necessarily all examples, the strain sensor comprises at least one fastener fastening the non-stretchable magnetic material to at least one of the plurality of layers.

In some but not necessarily all examples, the fastener comprises at least one of glue and one or more stitches.

In some but not necessarily all examples, the non-stretchable magnetic material comprises a rounded tip.

In some but not necessarily all examples, the non-stretchable magnetic material comprises at least one magnetic strip and/or at least one magnetic fibre.

In some but not necessarily all examples, the stretchable substrate comprises a first non-magnetic, non-conductive layer and a second non-magnetic, non-conductive layer, and the first non-magnetic, non-conductive layer and the second non-magnetic, non-conductive layer are the outer layers of the substrate.

According to various, but not necessarily all, embodiments there is provided an apparatus comprising:

at least one processor; and
at least one memory including computer program code;
the at least one memory and the computer program code configured to, with the at least one processor, cause the apparatus at least to perform:

measuring the inductance of a strain sensor, the strain sensor comprising:
a stretchable substrate comprising a plurality of layers;
non-stretchable magnetic material fastened to at least one of the plurality of layers; and
at least one coil of conductive thread wound through the substrate to mechanically interconnect the plurality of layers, wherein at least a portion of the at least one coil of conductive thread is located around at least a portion of the non-stretchable magnetic material; and the computer program code configured to, with the at least one processor, cause the apparatus to perform:
determining a change in the inductance of the strain sensor.

In some but not necessarily all examples, the computer program code is configured to, with the at least one processor, cause the apparatus to perform: determining at least one physiological measurement of a subject based, at least in part, on the determined change in inductance.

In some but not necessarily all examples, the at least one physiological measurement comprises at least one of breathing rate, heart rate, heart rate variability, blood pressure and posturing of a body part.

In some but not necessarily all examples, determining a change in the inductance of the strain sensor comprises monitoring a variation of the inductance of the strain sensor over time.

In some but not necessarily all examples, the computer program code is configured to, with the at least one processor, cause the apparatus to perform causing provision of at least one output based on, at least in part, the determined change in the inductance of the strain sensor.

In some but not necessarily all examples, the strain sensor comprises at least one fastener fastening the non-stretchable magnetic material to at least one of the plurality of layers.

In some but not necessarily all examples, the fastener comprises at least one of glue and one or more stitches.

In some but not necessarily all examples, the non-stretchable magnetic material comprises a rounded tip.

In some but not necessarily all examples, the non-stretchable magnetic material comprises at least one magnetic strip and/or at least one magnetic fibre.

In some but not necessarily all examples, the stretchable substrate comprises a first non-magnetic, non-conductive layer and a second non-magnetic, non-conductive layer, and the first non-magnetic, non-conductive layer and the second non-magnetic, non-conductive layer are the outer layers of the substrate.

In some but not necessarily all examples the apparatus and/or strain sensor is comprised in a wearable electronic device.

According to various, but not necessarily all, embodiments there is provided examples as claimed in the appended claims.

BRIEF DESCRIPTION

Some example embodiments will now be described with reference to the accompanying drawings in which:

FIG. 1 shows an example embodiment of the subject matter described herein;
FIGS. 2A and 2B show other example embodiments of the subject matter described herein;
FIGS. 3A and 3B show other example embodiments of the subject matter described herein;
FIGS. 4A and 4B show other example embodiments of the subject matter described herein;
FIGS. 5A and 5B show other example embodiments of the subject matter described herein;
FIGS. 6A and 6B show other example embodiments of the subject matter described herein;

FIG. 7 shows another example embodiment of the subject matter described herein;

FIGS. 8A and 8B show other example embodiments of the subject matter described herein;

FIG. 9 shows another example embodiment of the subject matter described herein; and

FIG. 10 shows another example embodiment of the subject matter described herein.

DETAILED DESCRIPTION

Examples of the disclosure relate to a strain sensor 10 and apparatuses, methods and computer programs for using a strain sensor 10.

In examples, the strain sensor 10 comprises a stretchable substrate 12 comprising a plurality of layers 14; non-stretchable magnetic material 16 fastened to at least one of the plurality of layers 14; and at least one coil of conductive thread 18 wound through the substrate 12 to mechanically interconnect the plurality of layers 14, wherein at least a portion of the at least one coil of conductive thread 18 is located around at least a portion of the non-stretchable magnetic material 16.

The strain sensor 10 is based on the functionality of an inductor. In examples, the strain sensor 10 can be considered an inductive-based strain sensor 10 or an inductive coupling-based strain sensor 10.

The use of one or more coils of conductive thread 18 wound through a stretchable multi-layer substrate 12 allows, for example, mechanical interconnection of stretchable materials, such as suitable fabrics or textiles, and at the same time having electronic functionality.

In examples, the stretchable substrate 12 can be formed from fabric or textile and at least one coil of conductive thread 18 can be used to attach together, for example, different parts or a wearable electronic device 52.

The strain sensor 10 can be small enough and light enough so that a wearable electronic device 52 comprising the strain sensor 10 is still comfortable for a subject to wear.

The strain sensor 10 can be compact, comfortable, stretchable and washable allowing such a strain sensor 10 to be used in wearable electronic devices 52.

Such a strain sensor 10 can easily be sealed by encapsulation to provide further water and/or solvent agent resistance.

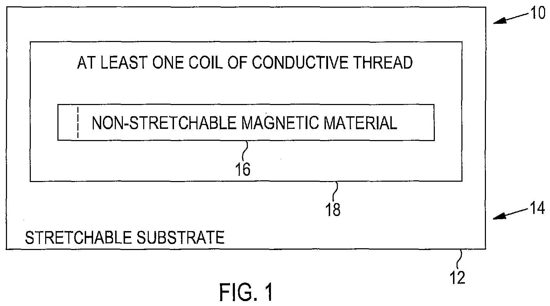

FIG. 1 schematically illustrates an example of a strain sensor 10.

Various features referred to in relation to FIG. 1 can be found in the other figures.

In FIG. 1 the strain sensor 10 comprises a stretchable substrate 12 comprising a plurality of layers 14, non-stretchable magnetic material 16 fastened to at least one of the plurality of layers 14 and at least one coil of conductive thread 18.

In the example of FIG. 1, the at least one coil of conductive thread 18 is wound through the stretchable substrate 12 to mechanically interconnect the plurality of layers 14. In examples the at least one coil of conductive thread 18 can be considered to attach, connect, interlock, join, link, fix and/or tie the plurality of layers 14.

However, in examples, the at least one coil of conductive thread 18 can be applied to and/or located in the stretchable substrate 12 in any suitable way to interconnect the plurality of layers 14.

For example, any suitable sewing, suturing or other similar method or technique can be used to locate the at least one coil of conductive thread 18 in the stretchable substrate 12.

At least a portion of the at least one coil of conductive thread 18 is located around at least a portion of the non-stretchable magnetic material 16.

In examples, the non-stretchable magnetic material 16 is located within the at least one coil of conductive thread 18.

The substrate 12 of the strain sensor 10 can comprise any suitable number and/or type of stretchable layers 14. For example, the layers 14 of the substrate 12 can comprise any suitable combination of material or materials.

Additionally, or alternatively, the layers 14 of the substrate 12 can be formed of any suitable shapes and/or sizes including the same or different shapes and/or sizes.

In examples, the layers 14 can comprise any suitable material or materials, for example, any suitable stretchable, flexible material and/or any suitable stretchable material for use in a wearable electronic garment.

In examples, at least one of the layers 14 is formed from fabric or textile. In some examples, all layers 14 of the substrate 12 are formed from fabric or textile.

According to some, but not necessarily all, examples the stretchable substrate 12 comprises a first non-magnetic, non-conductive layer 32 and a second non-magnetic, non-conductive layer 34, and wherein the first non-magnetic, non-conductive layer 32 of the second non-magnetic, non-conductive layer 34 are the outer layers of the stretchable substrate 12.

That is, the non-stretchable magnetic material 16 can be embedded between the two non-conductive, non-magnetic layers 32, 34.

Any suitable non-magnetic, non-conductive stretchable material can be used. For example, any suitable material that does not electrically conduct electricity and does not become magnetized.

In examples, any suitable non-magnetic, non-conductive stretchable textile or fabric such as polyester, nylon, cotton, lycra, bamboo, neoprene, rayon, acrylic, wool, silk and so on can be used.

In examples, the stretchable substrate 12 can comprise different portions of a garment that are mechanically interconnected by the at least one coil of conductive thread 18.

The non-stretchable magnetic material 16 can comprise any suitable non-stretchable magnetic material 16. For example, the magnetic material 16 can comprise any suitable non-stretchable magnetic material 16 to form a core of an inductor 44. The non-stretchable magnetic material 16 can have any suitable shape, size and/or form.

Any suitable magnetic/ferromagnetic material can be used. For example, elements containing iron such as ferrites, elements of composites containing nickel, cobalt or rare earth metals and so on.

In examples, the non-stretchable magnetic material 16 may be considered to be of fixed length.

In examples, the non-stretchable magnetic material 16 comprises at least one magnetic strip and/or at least one magnetic fibre.

A magnetic strip and a magnetic fibre can differ in shape and/or size. For example, a magnetic fibre can be narrower than a magnetic strip.

In some examples, the at least one coil of conductive thread 18 is wound around at least one magnetic strip and/or at least one magnetic fibre.

The non-stretchable magnetic material 16 is fastened to at least one of the plurality of layers 14.

The non-stretchable magnetic material 16 can be fastened, directly or indirectly, to at least one of the plurality of layers 14 in any suitable way.

In some, but not necessarily all, examples the strain sensor 10 comprises at least one fastener 20 fastening the non-stretchable magnetic material 16, directly or indirectly, to at least one of the plurality of layers 14.

Any suitable fastener 20 can be used. In examples, the fastener comprises at least one of glue 22 and one or more stitches 24.

The fastener(s) 20 can be located at any suitable location. In examples the fastener can be located to the side of the magnetic material 16 that is opposite compared to the stretching direction.

In examples the fastener(s) 20 can be located on the side of the magnetic material 16 that is the same as the stretching direction.

In examples, the non-stretchable magnetic material 16 can be considered to be fixed, attached, secured, tied, glued, stitched to, and/or constrained to at least one layer 14 of the stretchable subject 12.

In examples, the fastener 20 can be considered to be a fixing, attachment, constraint, tie, gluing, stitching and so on.

In examples, the non-stretchable magnetic material 16 is fastened to at least one of the plurality of layers 14 to allow the stretchable substrate 12, and the at least one coil of conductive thread 18 to move relative to the non-stretchable magnetic material 16 when the strain sensor 10 is put under strain.

In some examples, the non-stretchable magnetic material 16 is shaped to facilitate repeated movement of the material of the substrate 12 and the at least one coil of conductive thread 18 relative to the magnetic material 16.

For example, the non-stretchable magnetic material can comprise a rounded tip 26.

In examples the stretchable substrate 12 is stretchable relative to the non-stretchable magnetic material 16.

In examples the stretchable substrate 12 stretches under the application of a force under which the non-stretchable magnetic material 16 does not stretch or does not significantly stretch.

In examples the stretchable substrate 12 is configured to stretch under application of a force and the non-stretchable magnetic material is configured not to stretch, or not to significantly stretch, under application of that force.

In examples the stretchable substrate 12 is configured to stretch under application of a range of forces that will, in normal use, be applied to the strain sensor 10 and the non-stretchable magnetic material 16 is configured not to stretch, or not to significantly stretch, under application of that range of forces.

For example, the stretchable substrate 12 can be configured to stretch under forces applied to a strain sensor as part of a wearable electronic device and the non-stretchable magnetic material 16 can be configured not to stretch, or not to significantly stretch, under application of such forces.

Additionally or alternatively the stretchable substrate 12 can be configured to stretch under application of forces produced in measurement of at least one physiological measurement and the non-stretchable magnetic material 16 can be configured not to stretch, or not to significantly stretch, under the application of such forces.

In examples the stretchable substrate 12 is configured to stretch under application of forces in the intended measurement range of the strain sensor 10 and the non-stretchable magnetic material 16 is configured not to stretch, or not significantly stretch, under the application of those forces.

In examples the stretchable substrate 12 is stretchable as it is configured to stretch, in use, compared to the non-stretchable magnetic material 16 to allow movement of the at least one coil of conductive thread 18 relative to the non-stretchable magnetic material 16.

The at least one coil of conductive thread 18 can have any suitable form and/or shape and/or size. For example, the at least one coil of conductive thread 18 can have any suitable form and/or shape and/or size to form the windings of an inductor.

In examples, the at least one coil of conductive thread 18 can comprise any suitable number and/or size and/or pattern of turns or loops. For example, the number of turns in the coil 18 can be determined for or based on the sensitivity that the strain sensor 10 is to have.

In examples, the at least one coil of conductive thread 18 can be considered a spiral structure and/or helical structure and/or helix and/or spiral of conductive thread 18.

The conductive thread can comprise any suitable material or materials. In examples, the conductive thread comprises an outer insulating layer.

The strain sensor 10 can comprise any suitable number of coils of conductive thread 18.

For example, the strain sensor 10 can comprise a plurality of coils of conductive thread 18. In examples, the plurality of coils of conductive thread 18 can be the same or different.

The strain sensor 10 can be used wherever a strain sensor is required within an electronic device. For example, the strain sensor 10 could be used within a device for determining at least one physiological measurement of a subject. In examples, the strain sensor 10 can be used in determining a measurement of at least one of breathing rate, heart rate, heart rate variability, blood pressure and posturing of a body part.

Examples of the disclosure provide, for example, for a strain sensor 10 in wearable electronic devices being flexible, comfortable, water and sweat resistant/proof and/or washable.

The performance of the strain sensor 10, such as the sensitivity of the strain sensor 10, can be tuned by, for example, controlling the shape and number of loops in the at least one coil of conductive thread 18, which can be set by the stitching or suturing width.

Additionally or alternatively, the performance of the strain sensor 10, such as the sensitivity of the strain sensor 10, can be tuned by, for example, controlling the form and/or size of the magnetic material and/or the total component length.

Furthermore, the strain sensor 10 performance can be controlled by selection of the materials used in the sensor 10, for example by tuning the mechanical properties of the materials involved.

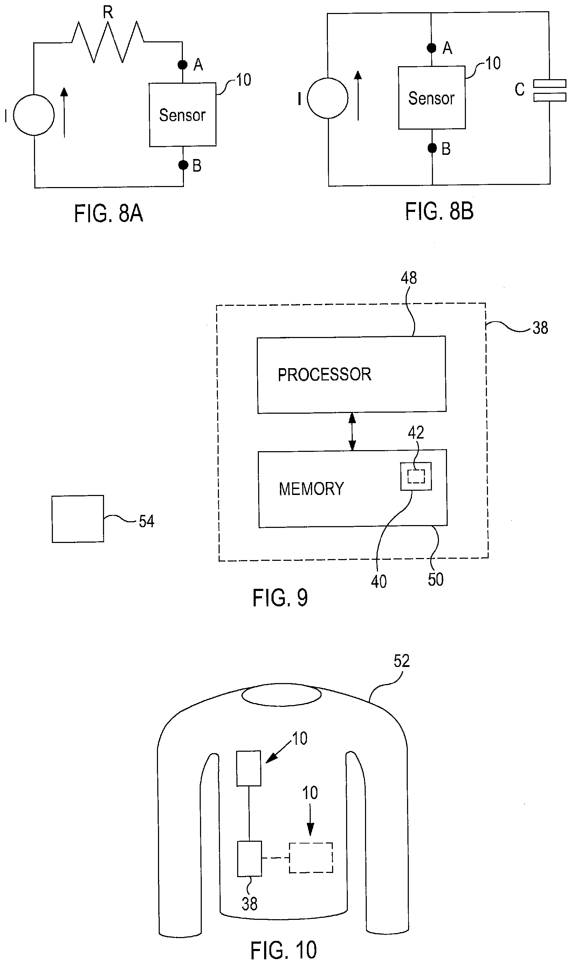

In examples, there is provided a wearable electronic device 52 comprising a strain sensor 10 as described herein. See, for example, FIG. 10.

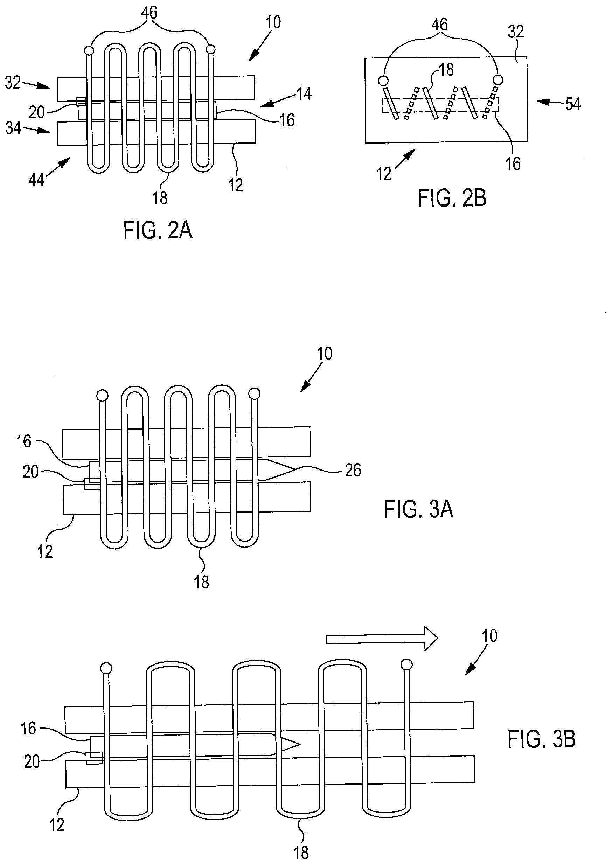

FIGS. 2A and 2B illustrates an example of a strain sensor 10. One or more elements of the strain sensor 10 can be as described in relation to FIG. 1.

Fig. 2A illustrates a cross-section of the strain sensor 10 and FIG 2B illustrates a top view of the strain sensor 10.

The strain sensor 10 comprises non-stretchable magnetic material 16 embedded between a first non-magnetic, non-conductive layer 32 and a second non-magnetic, non-conductive layer 34. The first and second non-magnetic, non-conductive layers 32, 34 are stretchable.

That is, in the example of FIG. 2A, the first and second non-magnetic, non-conductive layers 32, 34 are the outer layers of the substrate 12.

As illustrated in the example of FIG. 2A the coil of conductive thread 18 is wound through the layers 14 of the stretchable substrate 12 mechanically interconnecting the layers 14 of the stretchable substrate 12.

In the example of FIG. 2A, for the sake of clarity, the conductive thread is illustrated as extending beyond the upper and lower surfaces of the stretchable substrate 12. However, in examples, the conductive thread 18 can extend by any suitable amount and in some examples can be in contact with the upper and/or lower surfaces of the stretchable substrate 12.

The coil of conductive thread 18 is wound around the non-stretchable magnetic material 16 which provides the magnetic coil for the inductor 34.

This can be seen more clearly in the example of FIG. 2B.

The strain sensor 10 comprises connectors 46. The connectors 46 are configured to connect the strain sensor 10 to other circuitry components.

Any suitable connectors 46 formed using any suitable method can be used.

For example, connectors 46 can be obtained by making three-dimensional thread knots or using conductive folding crimps and so on.

The non-stretchable magnetic material 16 is fastened to the first non-magnetic, non-conductive layer 32 in the example of FIG. 2A.

In the illustrated example, the strain sensor 10 comprises a fastener 20 fastening the non-stretchable magnetic material 16 to the first non-magnetic, non-conductive layer 32.

In the example of FIG. 2A the fastener 20 is at the left end of the magnetic material 16.

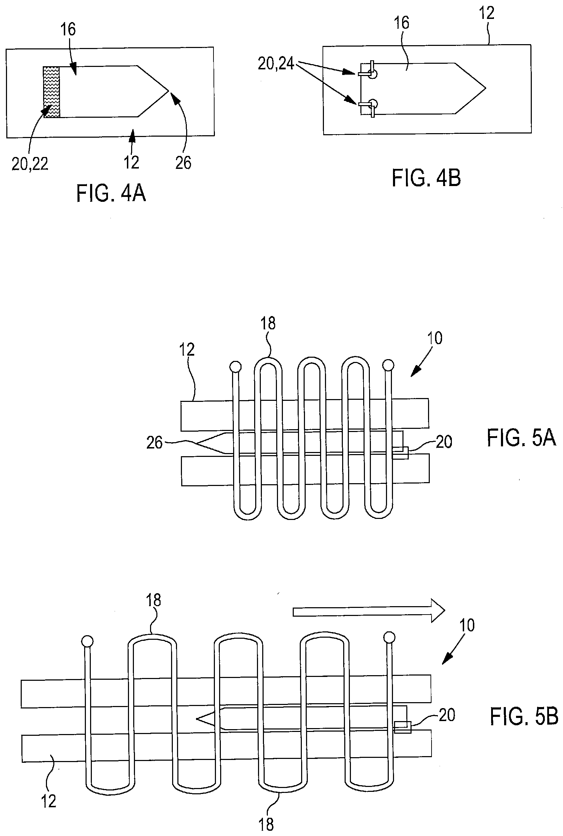

Any suitable fastener can be used. See, for example, FIGS. 4A and 4B.

In examples, any suitable number of fasteners 20 at any suitable number of location(s) on the non-stretchable magnetic material 16 can be used in any suitable combination.

The top view illustrated in the example of FIG. 2B illustrates the coil of conductive thread 18.

The portions of the conductive thread 18 on the upper portion are shown as a solid line and the portions of the conductive thread 18 on the lower portion are shown as dotted lines.

Although the coil of conductive thread 18 is illustrated as having a particular form in the example of FIGS. 2A and 2B, in examples any suitable form can be used.

For example, any suitable size or pattern of turns or loops of coil of conductive thread 18 can be used based on the functionality that the strain sensor 10 is to be configured to provide.

For example, the sensing range of the strain sensor 10 can be tuned according to the intended application.

In examples, if a large strain is to be measured, a longer strain sensor 10 can be used. The length of the strain sensor 10 can, in examples, be arbitrarily long and therefore any suitable length of strain sensor 10 can be used.

In examples where measurements of small strains are to be made a shorter strain sensor 10 can be used comprising a larger number of turns or coils of conductive thread 18 to improve the sensitivity of the strain sensor 10.

In the top view of FIG. 2B the non-stretchable magnetic material 16 is illustrated in a dashed line as it lies below the non-magnetic, non-conductive layer 32.

As can be seen in the examples of FIGS. 2A and 2B the coil of conductive thread 18 passes through the stretchable substrate 12 outside the area comprising the non-stretchable magnetic material 16.

As the non-stretchable magnetic material 16 is fastened to the first non-magnetic, non-conductive layer 32 the layers 14 of the substrate 12 will stretch and move relative to the non-stretchable magnetic material 16 when a stretching force or strain is applied to the strain sensor 10.

Accordingly, the application of a force or strain to the stretchable substrate 12 of the strain sensor 10 will cause the coil of conductive thread 18 to move relative to the non-stretchable magnetic material 16 causing a change in inductance of the strain sensor 10. See, for example, FIGS. 3A, 3B, 5A and 5B.

FIGS. 3A and 3B illustrate an example of use of a strain sensor 10. One or more elements of the strain sensor 10 illustrated in FIGS. 3A and 3B can be as described in relation to FIG. 1.

FIG. 3A illustrates a cross-section of a strain sensor 10 in a rest or unstrained or unstretched state.

The strain sensor 10 illustrated in the example of FIG. 3A is similar to the strain sensor illustrated in the example of FIGS. 2A and 2B. however, in the example of FIG. 3A the non-stretchable magnetic material 16 is fastened to the second non-magnetic, non-conductive layer 34 of the fastener 20.

In the example of FIG. 3A the non-stretchable magnetic material 16 also comprises a rounded tip 26. In the example of FIG. 3A, the rounded tip 26 is on the right side of non-stretchable magnetic material 16.

FIG. 3B illustrates the strain sensor 10 in a stretched or strained or expanded state.

In the example of FIG. 3B a force or strain is applied to the strain sensor 10 to the right as illustrated by the arrow pointing to the right in Fig. 3B.

The application of the force or strain causes the stretchable substrate 12 to stretch.

As the non-stretchable magnetic material 16 is fastened to a layer 14 of the stretchable substrate 12, in examples, it does not move with the application of force and also does not stretch.

Accordingly, the application of force or strain to the strain sensor 10 causes the coil of conductive thread 18 wound through the stretchable substrate 12 to move relative to the non-stretchable magnetic material 16.

As is illustrated in the example of FIG. 3B this causes one or more coils or loops of the conductive thread 18 to move away from the non-stretchable magnetic material 16.

As the strain or force applied to the strain sensor 10 increases, the stretchable substrate 12 will further stretch moving the at least one coil of conductive thread 18 further relative to the non-stretchable magnetic material 16 and therefore further affecting the inductance value of the strain sensor 10.

Therefore, the applied force or strain increases the length of the coil of conductive thread 18 without affecting the magnetic core length. As a result, a smaller number of conductive loops are wrapped around the magnetic core, causing a change in the inductance of the strain sensor 10 under strain. The inductance level can then be used as a measurement of strain.

Accordingly, as illustrated in the example of FIGS. 3A and 3B, the strain sensor 10 can be considered to have a first state in which the strain sensor 10 has a first inductance value and a second state in which the strain sensor 10 has a second, different inductance value.

In examples, the first state can be considered a relaxed or rest or unstrained state and the second state can be considered a strained or stretched or stressed state.

The rounded tip 26 of the non-stretchable magnetic material assists in the repeated movement of the strain sensor 10 between the two states.

FIGS. 4A and 4B illustrate examples of fasteners 20 for fastening non-stretchable magnetic material 16 to at least one layer of the stretchable substrate 12.

One or more elements illustrated in Figs. 4A and/or 4B can be as described in relation to FIG. 1.

In the example of FIG. 4A a top view of part of the strain sensor 10 is illustrated.

In the example of FIG. 4A, the stretchable substrate 12 is illustrated and the non-stretchable magnetic material 16 comprising a rounded tip 26 is also illustrated.

In the example of FIG. 4A a portion of the non-stretchable magnetic material 16 is glued to the stretchable substrate 12.

That is, in the example of FIG. 4A a portion of the non-stretchable magnetic material 16 is fixed/attached/constrained to the stretchable substrate 12 by glue 22.

This is illustrated in the example of FIG. 4A by the section of the magnetic material marked by wavy lines.

Any suitable glue can be used. For example, any glue that is water resistant and washable can be used. In examples the glue may comprise a fabric glue containing latex and/or a silicone-based glue and so on.

The example of FIG. 4B is similar to the example illustrated in FIG. 4A.

However, in the example of FIG. 4B the non-stretchable magnetic material 16 is fastened to the stretchable substrate 12 by a plurality of stitches 24.

In the example of FIG. 4B the non-stretchable magnetic material 16 comprises a plurality of holes to facilitate the application of the stitches 24 to fasten/attach the non-stretchable magnetic material 16 to the stretchable substrate 12.

However, in examples, any suitable method for using one or more stitches 24 to fasten the non-stretchable magnetic material 16 to the substrate 12 can be used.

For example, the number and/or location of holes can be varied.

Although the examples of FIGS. 4A and 4B illustrate examples of fasteners 20 that can be used to fasten the non-stretchable magnetic material 16 to the substrate 12, in examples any suitable method for fastening/attaching/constraining the non-stretchable magnetic material 16 to/relative to the stretchable substrate 12 can be used.

FIGS. 5A and 5B illustrate an example of use of a strain sensor 10.

One or more elements of the strain sensor 10 can be as described in relation to FIG. 1.

The examples of FIGS. 5A and 5B are similar to the example illustrated in FIGS. 3A and 3B.

However, in the example of FIGS. 5A and 5B the fastener 20 is located on the right of the non-stretchable magnetic material 16.

Furthermore, in the example of FIGS. 5A and 5B the rounded tip 26 of the non-stretchable magnetic material 16 is located on the left side of the non-stretchable magnetic material 16.

In the example of FIG. 5B a force or strain is applied to the strain sensor 10 towards the right as indicated by the arrow pointing to the right in the example of FIG. 5B.

As can be seen in the example of FIG. 5B, when a force or strain towards the right is applied to the strain sensor 10 the non-stretchable magnetic material 16, fastened to the stretchable substrate 12, moves with the stretchable substrate 12 towards the right.

The non-stretchable magnetic material 16 is therefore moved away from one or more coils of the at least one coil of conductive thread 18 resulting in movement of the magnetic material 16 relative to the substrate 12 and one or more coils of conductive thread 18.

This results in one or more coils of the conductive thread 18 no longer being wound around the magnetic core. Accordingly, this causes in a change in inductance of the strain sensor 10.

In the example of FIG. 5B the shape of the left side of the magnetic material facilitates the loop insertion when the strain sensor 10 relaxes back towards the rest or unstrained state.

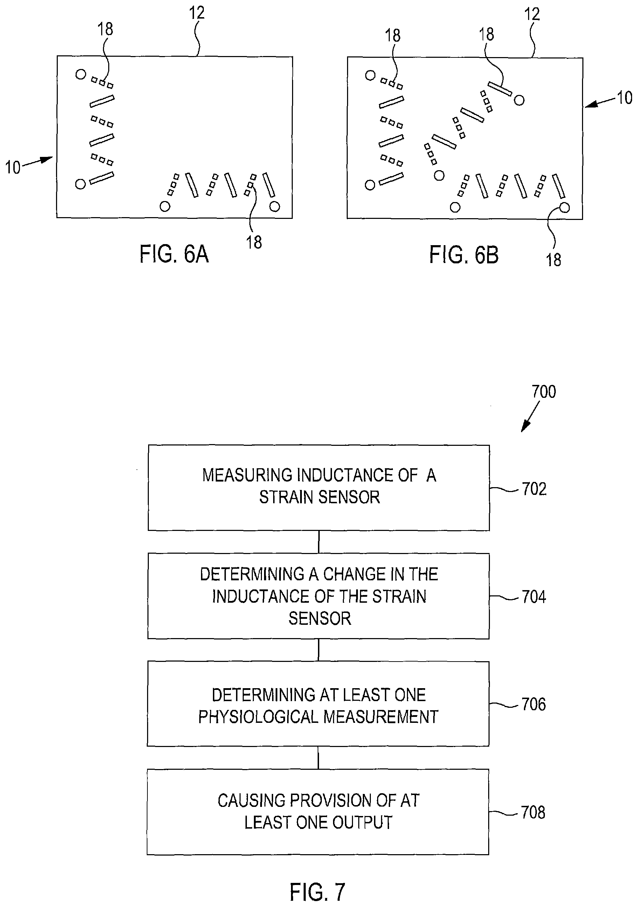

FIGS. 6A and 6B illustrate examples of stain sensors 10.

FIG. 6A illustrates an example of a bidimensional strain sensor 10 which enables a strain or stretching measurement to be performed along two dimensions. One or more elements of the strain sensor 10 can be as described in relation to FIG. 1.

In the example of FIG. 6A, the strain sensor 10 comprises two conductive coils of conductive thread 18 located substantially perpendicularly to each other.

In the example of FIG. 6A the strain sensor 10 comprises a coil of conductive thread 18 located horizontally on the stretchable substrate 12 and a coil of conductive thread 18 located vertically on the stretchable substrate 12.

In the example of FIG. 6A, the non-stretchable magnetic material 16 associated with the coils of conductive thread 18 is not illustrated in the top view.

However, within the coils of conductive thread 18 non-stretchable magnetic material 16 is present as described herein.

The strain sensor 10 illustrated in the example of FIG. 6A is configured to provide simultaneous strain measurement from two perpendicularly placed one-dimensional inductive strain sensors 10.

FIG. 6B illustrates an example of a strain sensor 10 comprising three coils of conductive thread 18.

The strain sensor 10 illustrated in the example of FIG. 6B is configured to measure more complex plain strain.

In the example of FIG. 6B two perpendicular coils of conductive thread 18 are present similar to FIG. 6A.

However, in the example of Fig. 6B a third coil of conductive thread 18 is located at substantially 45 degrees to the other coils of conductive thread 18.

As in the example of FIG. 6A the associated non-stretchable magnetic material 16 is not illustrated in the top view of FIG. 6B.

The configuration illustrated in the example of FIG. 6B allows for the measurement of plain strain, including shear strain measurements.

The bidirectional strain and plane strain configurations offer the advantage to simultaneously measure strains in two or more different directions using the same substrate 12 where the coils of conductive thread 18 can also be used to mechanically interconnect one or more layers 14 of the stretchable substrate 12.

In examples, the plain strain sensor 10 is useful to measure complex deformations, such as skin surface and shape changes around joints, knees, ankles, elbows and so on.

In examples, any suitable number and configuration of one-dimensional strain sensors 10 can be used.

FIG. 7 illustrates an example of the method 700. In the example of FIG. 7 the method 700 is a method of using a strain sensor 10.

In examples, the strain sensor 10 can be as described herein, for example as described in relation to at least one of FIGS. 1, 2A and 2B, 3A and 3B, 4A and 4B, 5A and 5B, 6A and 6B

At box 702 the inductance of a strain sensor 10 is measured, the strain sensor 10 comprising:

a stretchable substrate 12 comprising a plurality of layers 14;
non-stretchable magnetic material 16 fastened to at least one of the plurality of layers 14; and

at least one coil of conductive thread 18 wound through the substrate 12 to mechanically interconnect the plurality of layers 14, wherein at least a portion of the at least one coil of conductive thread 18 is located around at least a portion of the non-stretchable magnetic material 16.

Any suitable method for measuring the inductance of the strain sensor 10 can be used.

For example, any suitable means can be used, such as any suitable circuitry and/or controller and so on can be used to measure the inductance of the strain sensor 10.

For example, any suitable method for measuring the inductance between connectors 46 of the strain sensor 10 can be used. In examples the connectors 46 can be considered terminals.

A first example method of measuring the inductance of the strain sensor 10 is based on connecting the connectors 46 of the strain sensor 10 to a pulsed voltage source, such as an internal clock of a wearable electronic device 52 or any squared wave generated by driving electronics.

By applying the AC voltage, the AC current is measured. The inductance can be calculated starting from its definition:

$$v = L \cdot \frac{di(t)}{dt}$$

Where $v$ is the voltage applied between the connectors, $L$ is the inductance and $di(t)/dt$ is the derivative of the current over time.

The measured current waveform is a triangular wave, since the derivative of a triangular wave gives a square wave, which is the input voltage.

If a voltage is applied to an inductor, the current will increase linearly with time. Therefore, the inductance can be calculated from the slope of the resulting measured current wave as follows:

$$L = \frac{V_p \cdot T_{ON}}{I_p}$$

Where $V_p$ is the peak voltage applied to the circuit, $I_p$ is the measured peak current and $T_{on}$ is the ON time of the square wave.

A second example method for measuring the inductance of a strain sensor 10 is based on connecting a resistor in series with the strain sensor 10 and applying an AC current, see, for example, FIG. 8A.

In the example of FIG. 8A a strain sensor 10, as described herein, having connectors A and B is connected in series with a resistor, $R$, and an AC current source, $I$.

The AC voltage at the resistance terminals is then measured and the frequency of the current generator is adjusted to achieve a peak voltage, at connector A which is half of the peak input voltage on the other resistor terminal.

The determined frequency value and the value of the resistor, $R$, allows a calculation inductance, $L$, from the circuit illustrated in FIG. 8A.

The resistance voltage can be expressed as:

$$V_R = \frac{1}{2} \cdot V_{in} = I \cdot R = \frac{V_{in}}{R + j\omega L} \cdot R$$

Therefore:

$$\frac{R}{R + j\omega L} = \frac{1}{2}$$

Which gives:

$$L = \frac{\sqrt{3} \cdot R}{2\pi f}$$

Where $R$ is resistor value and $f$ is the determined frequency.

Another example method for measuring the inductance of the strain sensor 10 is based on using a resonant circuit, by connecting a capacitor in parallel with the strain sensor 10 and driving the circuit with an AC current generator. See, for example, FIG. 8B.

FIG. 8B illustrates a strain sensor 10 as described herein having connectors A and B connected in parallel with a capacitor $C$ and an AC current generator $I$.

The frequency of the AC generator can be adjusted to maximise the output at the strain sensor 10, which occurs at the resonant frequency. The inductance $L$ can then be calculated from the resonance expression:

$$\omega = \frac{1}{\sqrt{LC}}$$

The inductance $L$ can be then calculated as:

$$L = \frac{1}{(2\pi f)^2 \cdot C}$$

Where $f$ is the resonance frequency, $L$ is the sensor inductance and $C$ is the capacitor value.

Although various methods measuring the inductance of the strain sensor 10 have been described, in examples, any suitable method for measuring the inductance of the strain sensor 10 can be used.

Furthermore, any suitable means or controller for controlling measurement of an inductance of a strain sensor can be used. See, for example, FIG. 9.

In examples parasitic capacitances and/or resistive effects, such as the resistive effect of the at least one coil of conductive thread 18, can be included to increase the complexity of the read out circuit.

At block 704 a change in the inductance of the strain sensor 10 is determined.

In examples, any suitable method for determining a change in the inductance of the strain sensor 10 can be used.

For example, measured inductance values of the strain sensor 10 can be stored in a memory are compared to determine a change in the inductance of the strain sensor 10.

As described in relation to, for example, FIGS. 2A, 2B, 3A, 3B, 5A, 5B and 6A and 6B the determined change in inductance of the strain sensor 10 can be used as a measurement of strain on the strain sensor 10.

In examples, determining a change in the inductance of the strain sensor 10 comprises monitoring a variation of the inductance of the strain sensor 10 over time.

Any suitable method for monitoring a variation of the inductance of the strain sensor 10 over time can be used.

For example, measured values of inductance of the strain sensor 10 can be stored in a memory and monitored accordingly.

At block 706 at least one physiological measurement of a subject is determined based, at least in part, on the determined change of inductance of a strain sensor 10.

Any suitable method for determining at least one physiological measurement of a subject based, at least in part, on the determined change of inductance of a strain sensor 10 can be used.

For example, a strain sensor 10, as described herein, can be placed on a subject in proximity of any part of the body that is to be monitored. For example, a strain sensor 10, as described herein, can be placed in proximity of any vessel that expands in the body.

In examples, one or more strain sensors 10 can be located on the wrist, on a belt or in a shirt. However, any suitable part of a subject can be used such as legs, arms, neck and so on.

Lung expansions, for example, can be measured by placing one or more strain sensors 10 in a belt, shirt, or similar.

Whenever a heart rate or lung expansion occurs, for example, the substrate of the strain sensor 10 stretches which causes a change of inductance which can then be measured.

In examples, the at least one physiological measurement comprises at least one of breathing rate, heart rate, heart rate variability, blood pressure and posturing of a body part.

At block 708 provision of at least one output based on, at least in part, the determined change in the inductance of the strain sensor 10 is caused.

In examples, any suitable output based on, at least in part, the determined change in inductance of the strain sensor 10 can be provided.

For example, information on one or more physiological measurements can be provided to a user.

Additionally, or alternatively, one or more measurements of strain can be provided to a user.

In examples, one or more user interfaces can be controlled to provide an output determined, at least in part, on a determined change in inductance of the strain sensor 10.

The method 700 illustrated in FIG. 7 therefore comprises a method of using a strain sensor 10, the method comprising:

measuring the inductance of a strain sensor 10, the strain sensor 10 comprising:

    a stretchable substrate 12 comprising a plurality of layers 14;
    non-stretchable magnetic material 16 fastened to at least one of the plurality of layers 14; and
    at least one coil of conductive thread 18 wound through the substrate 12 to mechanically interconnect the plurality of layers 14, wherein at least a portion of the at least one coil of conductive thread 18 is located around at least a portion of the non-stretchable magnetic material 16; and the method comprising:
    determining a change in the inductance of the strain sensor 10.

In examples, one or more blocks of the method 700 may be altered or omitted. For example, one or more of blocks 706 and 708 can, in example, be omitted.

The method of FIG. 7 can be implemented using any suitable means. In examples, the method of FIG. 7 can be implemented using any suitable controller. See, for example, FIG. 9.

In examples, any suitable controller, such as the controller 38 of FIG. 9 can be used with any suitable circuitry, such as any suitable measurement circuitry, to perform the methods described herein.

The above described method may be enabled by a computer program comprising program instructions 42 for causing an apparatus 38 to perform a method as described herein. For example, a method comprising at least:

measuring the inductance of a strain sensor 10, the strain sensor 10 comprising:

    a stretchable substrate 12 comprising a plurality of layers 14;
    non-stretchable magnetic material 16 fastened to at least one of the plurality of layers 14; and
    at least one coil of conductive thread 18 wound through the substrate 12 to mechanically interconnect the plurality of layers 14, wherein at least a portion of the at least one coil of conductive thread 18 is located around at least a portion of the non-stretchable magnetic material 16; and the method comprising:

determining a change in the inductance of the strain sensor 10.

FIGS. 8A and 8B illustrate example circuitry for enabling measurement of inductance of a strain sensor 10 described herein.

See, for example, FIG. 7.

FIG. 9 illustrates an example of a controller 38. Implementation of a controller 38 may be as controller circuitry. The controller 38 may be implemented in hardware alone, have certain aspects in software including firmware alone or can be a combination of hardware and software (including firmware).

As illustrated in FIG. 9 the controller 38 may be implemented using instructions that enable hardware functionality, for example, by using executable instructions of a computer program 40 in a general-purpose or special-purpose processor 48 that may be stored on a computer readable storage medium (disk, memory etc) to be executed by such a processor 48.

The processor 48 is configured to read from and write to the memory 50. The processor 48 may also comprise an output interface via which data and/or commands are output by the processor 48 and an input interface via which data and/or commands are input to the processor 48.

The memory 50 stores a computer program 40 comprising computer program instructions (computer program code) that controls the operation of the apparatus 38 when loaded into the processor 48. The computer program instructions, of the computer program 40, provide the logic and routines that enables the apparatus to perform the methods illustrated in FIG. 7. The processor 48 by reading the memory 50 is able to load and execute the computer program 40.

The apparatus 38 therefore comprises:

at least one processor 48; and
at least one memory 50 including computer program code
the at least one memory 50 and the computer program code configured to, with the at least one processor 48, cause the apparatus 38 at least to perform:
A method of using a strain sensor, the method comprising:
measuring the inductance of a strain sensor, the strain sensor comprising:

a stretchable substrate comprising a plurality of layers;
non-stretchable magnetic material fastened to at least one of the plurality of layers; and
at least one coil of conductive thread wound through the substrate to mechanically interconnect the plurality of layers, wherein at least a portion of the at least one coil of conductive thread is located around at least a portion of the non-stretchable magnetic material; and the method comprising:
determining a change in the inductance of the strain sensor.

As illustrated in FIG. 9, the computer program 40 may arrive at the apparatus 38 via any suitable delivery mechanism 54. The delivery mechanism 54 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 40. The delivery mechanism may be a signal configured to reliably transfer the computer program 40. The apparatus 38 may propagate or transmit the computer program 40 as a computer data signal.

Computer program instructions 42 for causing an apparatus to perform at least the following or for performing at least the following:
A method of using a strain sensor, the method comprising:
measuring the inductance of a strain sensor, the strain sensor comprising:

a stretchable substrate comprising a plurality of layers;
non-stretchable magnetic material fastened to at least one of the plurality of layers; and
at least one coil of conductive thread wound through the substrate to mechanically interconnect the plurality of layers, wherein at least a portion of the at least one coil of conductive thread is located around at least a portion of the non-stretchable magnetic material; and the method comprising:
determining a change in the inductance of the strain sensor.

The computer program instructions 42 may be comprised in a computer program, a non-transitory computer readable medium, a computer program product, a machine readable medium. In some but not necessarily all examples, the computer program instructions 42 may be distributed over more than one computer program.

Although the memory 50 is illustrated as a single component/circuitry it may be implemented as one or more separate components/circuitry some or all of which may be integrated/removable and/or may provide permanent/semi-permanent/ dynamic/cached storage.

Although the processor 48 is illustrated as a single component/circuitry it may be implemented as one or more separate components/circuitry some or all of which may be integrated/removable. The processor 48 may be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' may refer to one or more or all of the following:

> (a) hardware-only circuitry implementations (such as implementations in only analog and/or digital circuitry) and
> (b) combinations of hardware circuits and software, such as (as applicable):
>
>> (i) a combination of analog and/or digital hardware circuit(s) with software/firmware and
>> (ii) any portions of hardware processor(s) with software (including digital signal processor(s)), software, and memory(ies) that work together to cause an apparatus, such as a mobile phone or server, to perform various functions and
>
> (c) hardware circuit(s) and or processor(s), such as a microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in the FIG. 3 can represent steps in a method and/or sections of code in the computer program 40. The illustration of a particular order to the blocks does not necessarily imply that there is a required or preferred order for the blocks and the order and arrangement of the block may be varied. Furthermore, it may be possible for some blocks to be omitted.

In examples the controller 38 provides an apparatus comprising:

> means for measuring the inductance of a strain sensor, the strain sensor comprising:
>
>> a stretchable substrate comprising a plurality of layers;
>> non-stretchable magnetic material fastened to at least one of the plurality of layers; and
>> at least one coil of conductive thread wound through the substrate to mechanically interconnect the plurality of layers, wherein at least a portion of the at least one coil of conductive thread is located around at least a portion of
>
> the non-stretchable magnetic material; and the apparatus comprising:
> means for determining a change in the inductance of the strain sensor.

In examples there is a provided a system comprising a strain sensor 10 as described herein and a controller 38 as described herein.

For example, the strain sensor 10 can be as described in relation to at least one of FIGS 1, 2A, 2B, 3A, 3B, 5A, 5B, 6A and 6B.

In examples the controller 38 can be as described in relation to FIG. 10.

Therefore, in examples, there is provided a system comprising:

> a strain sensor comprising:
>
>> a stretchable substrate comprising a plurality of layers;
>> non-stretchable magnetic material fastened to at least one of the plurality of layers; and
>> at least one coil of conductive thread wound through the substrate to mechanically interconnect the plurality of layers, wherein at least a portion of the at least one coil of conductive thread is located around at least a portion of the non-stretchable magnetic material; and
>
> an apparatus comprising:
>
>> means for measuring the inductance of the strain sensor; and
>> means for determining a change in the inductance of the strain sensor.

In examples the apparatus comprises means for determining at least one physiological measurement of a subject based, at least in part, on the determined change in inductance.

FIG. 10 illustrates an example of a wearable electronic device 52 comprising at least one strain sensor 10. In the illustrated example the strain sensor 10 is as described in at last one of FIGS.1 to 6.

In the example of FIG. 10 the wearable electronic device 52 comprises a garment comprising one or more strain sensors 10. The garment comprises an item of clothing which is arranged to be worn by a subject. In the example of FIG. 10 the garment comprises a shirt.

Other items of clothing could be used in other examples of the disclosure. For instance, the item of clothing could comprise a strap which could be attached to any suitable part of the subject's body, or any other suitable article of clothing such as trousers, sleeve, belt, jacket and similar.

In examples, the garment comprises an item of clothing which is arranged to be attached to and/or worn by an animal.

In such examples, the use of one or more strain sensors 10 in garments and/or items of clothing arranged to be attached to and/or worn by an animal can provide for monitoring of one or more physiological parameters of the animal.

In the example of FIG. 10 the one or more strain sensors 10 are coupled to other circuitry components. The other circuitry components could be any suitable circuitry components but in the example of FIG. 10 a controller 38 is illustrated. The controller 38 can be as described in relation to FIG. 9.

Where a structural feature has been described, it may be replaced by means for performing one or more of the functions of the structural feature whether that function or those functions are explicitly or implicitly described.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one.." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although embodiments have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain embodiments, those features may also be present in other embodiments whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer and exclusive meaning.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

The scope of protection of the invention is defined by the appended claims.

**Claims**

**1.** A strain sensor (10) comprising:

a stretchable substrate (12) comprising a plurality of layers (14);

non-stretchable magnetic material (16) fastened to at least one of the plurality of layers (14); and

at least one coil of conductive thread (18) wound through the substrate (12) to mechanically interconnect the plurality of layers (14), wherein at least a portion of the at least one coil of conductive thread (18) is located around at least a portion of the non-stretchable magnetic material (16).

2. A strain sensor (10) as claimed in claim 1, comprising at least one fastener fastening the non-stretchable magnetic material (16) to at least one of the plurality of layers (14).

3. A strain sensor (10) as claimed in claim 2, wherein the fastener comprises at least one of glue and one or more stitches.

4. A strain sensor (10) as claimed in any preceding claim, wherein the non-stretchable magnetic material (16) comprises a rounded tip.

5. A strain sensor (10) as claimed in any preceding claim, wherein the non-stretchable magnetic material (16) comprises at least one magnetic strip and/or at least one magnetic fibre.

6. A strain sensor (10) as claimed in any preceding claim, wherein the stretchable substrate (12) comprises a first non-magnetic, non-conductive layer and a second non-magnetic, non-conductive layer, and wherein the first non-magnetic, non-conductive layer and the second non-magnetic, non-conductive layer are the outer layers of the substrate (12).

7. A wearable electronic device comprising a strain sensor (10) as claimed in at least one of claims 1 to 6.

8. A method of using a strain sensor (10), the method comprising:

measuring the inductance of a strain sensor (10) as claimed in at least one of claims 1 to 6; and determining a change in the inductance of the strain sensor (10).

9. A method as claimed in claim 8, comprising determining at least one physiological measurement of a subject based, at least in part, on the determined change in inductance.

10. A method as claimed in claim 9, wherein the at least one physiological measurement comprises at least one of breathing rate, heart rate, heart rate variability, blood pressure and posturing of a body part.

11. A method as claimed in any of claims 8 to 10 wherein determining a change in the inductance of the strain sensor (10) comprises monitoring a variation of the inductance of the strain sensor (10) over time.

12. A method as claimed in any of claims 8 to 11, the method comprising:
causing provision of at least one output based on, at least in part, the determined change in the inductance of the strain sensor (10).

13. A system comprising:

a strain sensor (10)as claimed in at least one of claims 1 to 6; and
an apparatus comprising:

means for measuring the inductance of the strain sensor (10); and
means for determining a change in the inductance of the strain sensor.

14. A system as claimed in claim 13, the apparatus comprising means for determining at least one physiological measurement of a subject based, at least in part, on the determined change in inductance.

15. A wearable electronic device comprising a system according to at least one of claims 13 and 14.

**Patentansprüche**

1. Dehnungssensor (10), der Folgendes umfasst:

ein dehnbares Substrat (12), das eine Vielzahl von Schichten (14) umfasst;
ein nicht dehnbares magnetisches Material (16), das an mindestens einer der Vielzahl von Schichten (14) befestigt ist; und
mindestens eine Spule eines leitfähigen Fadens (18), der durch das Substrat (12) gewickelt ist, um die Vielzahl von Schichten (14) mechanisch zu verbinden, wobei mindestens ein Abschnitt der mindestens einen Spule eines leitfähigen Fadens (18) um mindestens einen Abschnitt des nicht dehnbaren magnetischen Materials (16) positioniert ist.

2. Dehnungssensor (10) nach Anspruch 1, der mindestens ein Befestigungsmittel zum Befestigen des nicht dehnbaren magnetischen Materials (16) an mindestens einer der Vielzahl von Schichten (14) umfasst.

3. Dehnungssensor (10) nach Anspruch 2, wobei das Befestigungsmittel mindestens eines von einem Kleber und einem oder mehreren Knoten umfasst.

**4.** Dehnungssensor (10) nach einem der vorhergehenden Ansprüche, wobei das nicht dehnbare magnetische Material (16) eine abgerundete Spitze umfasst.

**5.** Dehnungssensor (10) nach einem der vorhergehenden Ansprüche, wobei das nicht dehnbare magnetische Material (16) mindestens einen magnetischen Streifen und/oder mindestens eine magnetische Faser umfasst.

**6.** Dehnungssensor (10) nach einem der vorhergehenden Ansprüche, wobei das dehnbare Substrat (12) eine erste nicht magnetische, nicht leitfähige Schicht und eine zweite nicht magnetische, nicht leitfähige Schicht umfasst, und wobei die erste nicht magnetische, nicht leitfähige Schicht und die zweite nicht magnetische, nicht leitfähige Schicht die äußeren Schichten des Substrats (12) sind.

**7.** Tragbare elektronische Vorrichtung, die einen Dehnungssensor (10) mindestens nach einem der Ansprüche 1 bis 6 umfasst.

**8.** Verfahren zum Verwenden eines Dehnungssensors (10), wobei das Verfahren Folgendes umfasst:

Messen der Induktanz eines Dehnungssensors (10) mindestens nach einem der Ansprüche 1 bis 6; und
Bestimmen einer Änderung der Induktanz des Dehnungssensors (10).

**9.** Verfahren nach Anspruch 8, das das Bestimmen von mindestens einer physiologischen Messung einer Person mindestens teilweise auf Basis der bestimmten Änderung der Induktanz umfasst.

**10.** Verfahren nach Anspruch 9, wobei die mindestens eine physiologische Messung mindestens eines von einer Atemfrequenz, einer Herzfrequenz, einer Herzfrequenzvariabilität, eines Blutdrucks und einer Haltung eines Körperteils umfasst.

**11.** Verfahren nach einem der Ansprüche 8 bis 10, wobei das Bestimmen einer Änderung der Induktanz des Dehnungssensors (10) das Überwachen einer Variation der Induktanz des Dehnungssensors (10) über die Zeit umfasst.

**12.** Verfahren nach einem der Ansprüche 8 bis 11, wobei das Verfahren Folgendes umfasst:
Bewirken der Bereitstellung von mindestens einer Ausgabe mindestens teilweise auf Basis der bestimmten Änderung der Induktanz des Dehnungssensors (10).

**13.** System, das Folgendes umfasst:

einen Dehnungssensor (10) mindestens nach einem der Ansprüche 1 bis 6; und
eine Vorrichtung, die Folgendes umfasst:

Mittel zum Messen der Induktanz des Dehnungssensors (10); und
Mittel zum Bestimmen einer Änderung der Induktanz des Dehnungssensors.

**14.** System nach Anspruch 13, wobei die Vorrichtung Mittel zum Bestimmen von mindestens einer physiologischen Messung einer Person mindestens teilweise auf Basis der bestimmten Änderung der Induktanz umfasst.

**15.** Tragbare elektronische Vorrichtung, die ein System mindestens gemäß einem der Ansprüche 13 und 14 umfasst.

## Revendications

**1.** Capteur de contrainte (10) comprenant :

un substrat (12) étirable comprenant une pluralité de couches (14);
un matériau magnétique (16) non étirable fixé à au moins l'une de la pluralité de couches (14); et
au moins une bobine d'un filetage conducteur (18) enroulée à travers le substrat (12) pour interconnecter mécaniquement la pluralité de couches (14), dans lequel au moins une partie de la au moins une bobine d'un filetage conducteur (18) est située autour d'au moins une partie du matériau magnétique (16) non étirable.

**2.** Capteur de contrainte (10) selon la revendication 1, comprenant au moins une pièce de fixation fixant le matériau magnétique (16) non étirable à au moins l'une de la pluralité de couches (14).

**3.** Capteur de contrainte (10) selon la revendication 2, dans lequel la pièce de fixation comprend au moins l'un parmi une colle et un ou plusieurs points de liage.

**4.** Capteur de contrainte (10) selon l'une quelconque des revendications précédentes, dans lequel le matériau magnétique (16) non étirable comprend une pointe arrondie.

**5.** Capteur de contrainte (10) selon l'une quelconque des revendications précédentes, dans lequel le matériau magnétique (16) non étirable comprend au moins une bande magnétique et/ou au moins une fibre magnétique.

6. Capteur de contrainte (10) selon l'une quelconque des revendications précédentes, dans lequel le substrat (12) étirable comprend une première couche non magnétique, non conductrice et une seconde couche non magnétique, non conductrice, et dans lequel la première couche non magnétique, non conductrice et la seconde couche non magnétique, non conductrice sont les couches extérieures du substrat (12).

7. Dispositif électronique portable comprenant un capteur de contrainte (10) selon au moins l'une des revendications 1 à 6.

8. Procédé d'utilisation d'un capteur de contrainte (10), le procédé comprenant :

la mesure de l'inductance d'un capteur de contrainte (10) selon au moins l'une des revendications 1 à 6; et
la détermination d'un changement dans l'inductance du capteur de contrainte (10).

9. Procédé selon la revendication 8, comprenant la détermination d'au moins une mesure physiologique d'un sujet sur la base, au moins partiellement, du changement déterminé en inductance.

10. Procédé selon la revendication 9, dans lequel la au moins une mesure physiologique comprend au moins l'un parmi un rythme de la respiration, une fréquence cardiaque, une variabilité de fréquence cardiaque, une pression artérielle et une posture d'une partie de corps.

11. Procédé selon l'une quelconque des revendications 8 à 10, dans lequel la détermination d'un changement dans l'inductance du capteur de contrainte (10) comprend le pilotage d'une variation de l'inductance du capteur de contrainte (10) au fil du temps.

12. Procédé selon l'une quelconque des revendications 8 à 11, le procédé comprenant :
le fait de provoquer la fourniture d'au moins une sortie sur la base, au moins partiellement, du changement déterminé dans l'inductance du capteur de contrainte (10).

13. Système comprenant :

un capteur de contrainte (10) selon au moins l'une des revendications 1 à 6; et
un appareil comprenant :

un moyen pour mesurer l'inductance du capteur de contrainte (10) ; et

un moyen pour déterminer un changement dans l'inductance du capteur de contrainte.

14. Système selon la revendication 13, l'appareil comprenant un moyen pour déterminer au moins une mesure physiologique d'un sujet sur la base, au moins partiellement, du changement déterminé en inductance.

15. Dispositif électronique portable comprenant un système selon au moins l'une des revendications 13 et 14.

AT LEAST ONE COIL OF CONDUCTIVE THREAD

NON-STRETCHABLE MAGNETIC MATERIAL

16

18

10

14

STRETCHABLE SUBSTRATE

12

FIG. 1

FIG. 2A

FIG. 2B

FIG. 3A

FIG. 3B

FIG. 4A

FIG. 4B

FIG. 5A

FIG. 5B

FIG. 6A          FIG. 6B

700

| MEASURING INDUCTANCE OF A STRAIN SENSOR | 702 |

| DETERMINING A CHANGE IN THE INDUCTANCE OF THE STRAIN SENSOR | 704 |

| DETERMINING AT LEAST ONE PHYSIOLOGICAL MEASUREMENT | 706 |

| CAUSING PROVISION OF AT LEAST ONE OUTPUT | 708 |

FIG. 7

FIG. 8A

FIG. 8B

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4890084 A